(19) Europäisches Patentamt
European Patent Office
Office européen des brevets

(11) EP 0 643 702 B1

(12) **EUROPEAN PATENT SPECIFICATION**

(45) Date of publication and mention of the grant of the patent:
**02.01.1997 Bulletin 1997/01**

(21) Application number: **93911316.3**

(22) Date of filing: **14.05.1993**

(51) Int Cl.6: **C07D 239/38**, C07D 239/56, C08K 5/44, C08L 21/00

(86) International application number:
**PCT/US93/04593**

(87) International publication number:
**WO 93/25538 (23.12.1993 Gazette 1993/30)**

# (54) 4-PYRIMIDINE SULFENAMIDES AND THEIR USE IN RUBBER

4-PYRIMIDIN SULFENAMIDE UND IHRE VERWENDUNG IN KAUTSCHUK

SULFENAMIDES DE LA 4-PYRIMIDINE ET LEUR UTILISATION DANS LE CAOUTCHOUC

(84) Designated Contracting States:
**AT BE CH DE DK ES FR GB GR IE IT LI LU MC NL PT SE**

(30) Priority: **05.06.1992 US 894677**
**04.05.1993 US 53972**

(43) Date of publication of application:
**22.03.1995 Bulletin 1995/12**

(73) Proprietor: **MONSANTO COMPANY**
**St. Louis Missouri 63167 (US)**

(72) Inventors:
- **LIN, Horng-Jau**
  **Wadsworth, OH 44281 (US)**
- **ROSTEK, Charles, John, Jr.**
  **Bentleyville, OH 44022 (US)**
- **SIKORA, David, John**
  **Hudson, OH 44236 (US)**

(74) Representative: **Ernst, Hubert et al**
**Monsanto Services International S.A./N.V.,**
**Patent Department,**
**Avenue de Tervuren 270-272,**
**Letter Box No. 21**
**1150 Bruxelles (BE)**

(56) References cited:
**FR-A- 2 126 730** **GB-A- 511 926**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### FIELD OF THE INVENTION

This invention relates to certain pyrimidine sulfenamides and to their use in rubber.

### BACKGROUND

A number of heterocyclic sulfenamides have been well known, as has been their use in the vulcanization of rubber. The best known and most widely used are based on benzothiazole. Thus, the benzothiazole sulfenamides, such as N-t-butyl-2-benzothiazole sulfenamide (TBBS) and N-cyclohexyl-2-benzothiazole sulfenamide (CBS) have become standard accelerators of vulcanization. Similarly, the thiol derivative, 2-mercaptobenzothiazole (MBT) and the disulfide derivative, 2,2'-benzothiazole disulfide (MBTS), are standards of the industry.

To a lesser degree, other N-heterocycles have been suggested as the basis for sulfenamides. For example, British Patent 795,174 describes a process for making a large variety of sulfonamide compounds for use as diuretics and antibacterial agents in which the sulfenamide equivalent is first made as an intermediate. Twenty-six different basic heterocycles are suggested, and a wide variety of substituents and fused ring variations are included, as well.

Similarly, British Patent 1,342,046 discloses a process for making heterocyclic sulfenamides, based on diazine, triazine and pyridine thiols, encompassing an unlimited number of possible compounds, suggested to be effective vulcanization accelerators for rubber.

Specific heterocyclic sulfenamides based on 2-mercaptopyrimidine are disclosed in British Patent 802,622 and in D'Amico, U. S. Patent 3,839,303.

### SUMMARY OF THE INVENTION

It has now been found that sulfenamides based on the 4-pyrimidyl moiety are particularly effective accelerators for the vulcanization of natural and synthetic rubber. Surprisingly, they are considerably more active than their isomeric counterparts (the 2-pyrimidine sulfenamides of British Patent 802,622 and U. S. Patent 3,839,303) in terms of cure rate and extent of cure (cure state). 4-Pyrimidine sulfenamides have been found to have superior accelerating effect on the vulcanization of natural and synthetic rubber, compared to the isomeric 2-pyrimidine sulfenamides.

The compounds of the present invention when utilized as accelerators for curing natural rubber, synthetic rubbers such as polybutadiene, EPDM or styrene-butadiene rubber, blends of rubbers such as natural rubber and polybutadiene, styrene-butadiene rubber and polybutadiene, or combinations thereof, result in improved cure rates as indicated by t90-t2 values, t25-t2 values and maximum rate of vulcanization (Vmax), better scorch delay, and higher extent of cure (cure efficiency) in comparison with traditional or conventional sulfenamide accelerators. Increased cure rates are very desirable since faster rates of production of rubber articles can be obtained. Molded rubber articles, such as tires, can thus be removed from the mold at earlier times without the risk of undercure. While it is generally possible to increase the cure rate of a rubber compound (up to a point) by using combinations of accelerators and/or higher levels of accelerators, these changes are often accompanied by unacceptable losses of scorch delay. Longer scorch delay is desirable to provide a longer time for the rubber article to be shaped and molded at processing temperatures before the onset of vulcanization. Higher extents of cure may negate the use of sulfur donors.

### BRIEF DESCRIPTION OF THE DRAWING

The drawing is a typical rheograph showing the parameters of the vulcanization reaction.

### DETAILED DESCRIPTION OF THE INVENTION

The compounds of the invention, which are used in rubber compositions to produce improved vulcanization behavior and/or improved vulcanizate properties, are based on 4-pyrimidine (4-(1,3-diazine)).

A general formula for the compounds of the invention is

PmSNRR'

Pm ≡ 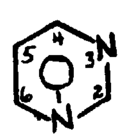

wherein Pm is 4-pyrimidyl, optionally substituted on the nucleus by one or more halogen atoms or lower alkyl, phenyl, lower alkoxy or hydroxyl groups; R is H or $C_{1-8}$ alkyl, $C_{3-8}$ cycloalkyl, phenyl, $C_{7-12}$ aralkyl or $C_{7-12}$ alkaryl; R' is H or R, or R and R' together with N form a heterocyclic ring.

The sulfenamide compounds of the invention all have a sulfenamide group attached at the 4 position, such that this sulfenamide group is based on a primary or secondary amine. The primary amines which can be used include $C_{1-8}$ alkyl amines such as methylamine, ethylamine, n-propylamine, isopropylamine, n-butylamine, sec-butylamine, isobutylamine, t-butylamine, n-amylamine, t-octylamine and the like;
$C_{3-8}$ cycloalkylamines such as cyclopropylamine, cyclohexylamine, cyclooctylamine and the like; phenylamine (aniline); $C_{7-12}$ aralkylamines such as benzylamine and the like; and $C_{7-12}$ alkarylamines such as p-t-butyl aniline and the like. Secondary amines include diisopropylamine, dicyclohexylamine and the like.

As indicated, one or more substituents can be present at the open positions on the pyrimidine ring, such as halogen atoms, or lower alkyl, lower alkoxy, phenyl or hydroxyl groups.

Preferred sulfenamides of the invention are those made from isopropylamine, t-butylamine or cyclohexylamine; and thus include N-isopropyl-4-pyrimidine sulfenamide, N-t-butyl-4-pyrimidine sulfenamide, N-cyclohexyl-4-pyrimidine sulfenamide and the like.

The sulfenamides of the invention can be prepared from the respective thiol or disulfide by treatment with amine in the presence of silver nitrate or an oxidizing agent such as sodium hypochlorite or oxygen.

The 4-pyrimidine sulfenamides of the present invention can be used as primary or auxiliary accelerators in the vulcanization of rubber. Generally any type of sulfur vulcanizable rubber can be utilized such as natural rubber, synthetic rubber, various blends of synthetic rubber and combinations thereof. Natural rubber is usually obtained from hevea Brasiliensis trees, and is generally grown in the tropics. Synthetic rubbers include those made from various dienes such as those having from 4 to 12 carbon atoms and preferably from 4 to 8 carbon atoms including 1,3-butadiene, isoprene, 2,3-dimethyl-1,3-butadiene, 2-methyl-1,3-pentadiene, 3,4-dimethyl-1,3-hexadiene, 4,5-diethyl-1,3-octadiene, phenyl-1,3-butadiene, pentadiene, hexadiene, octadiene, and the like. Synthetic rubbers also include copolymers made from the immediately above-noted dienes having from 4 to 12 carbon atoms with a vinyl substituted aromatic compound having from 8 to 20 carbon atoms such as styrene, alpha-methylstyrene, 4-n-propylstyrene, 4-t-butylstyrene, and the like, as well as copolymers made from the above dienes and acrylonitrile.

Another class of synthetic rubbers which can be utilized in the present invention are EPDM rubbers. These are polymers made from ethylene, propylene, and a minor proportion of a non-conjugated diene monomer such as ethylidenenorbornene, dicyclopentadiene, 1,4-hexadiene and the like. Butyl rubbers, which are copolymers from isobutylene and a minor proportion of isoprene, can be used, as well as their halogenated derivatives, such as chlorobutyl or bromobutyl rubber. Other sulfur vulcanizable rubbers known to the art and to the literature can also be utilized.

The rubber polymers made from conjugated dienes or copolymers of a conjugated diene or the vinyl substituted aromatic are preferably "elastomeric" materials, that is they conform, when vulcanized, to the definition of an elastomeric or rubber material found in ASTM D 1566.

As noted above, either natural rubber, one or more synthetic rubbers, that is either a single type of synthetic rubber or blends of two or more synthetic rubbers, as well as a blend of natural rubber and one or more synthetic rubbers can be cured utilizing one of the diazine compounds of the present invention as a primary accelerator. When utilized as a primary accelerator, the amount thereof is generally from about 0.1 to about 10 parts and preferably from about 0.2 to about 2.0 parts by weight per 100 parts by weight (phr) of the rubber polymer or blend. When the 4-pyrimidine sulfenamides of the invention are utilized as accelerators for curing rubber compounds, the natural or synthetic rubber compositions of the present invention generally contain other conventional compounding ingredients in conventional amounts, both of which are well known to the art and to the literature. Sulfur, in amounts of from 0.5 to 5 phr, is usually employed. Also, various fillers and reinforcing agents, such as clay, silica, and carbon black, can be utilized in amounts from 5 up to about 200 phr. Various oils, for example aromatic, naphthenic, or paraffinic, can be utilized to plasticize the rubber in amounts from 5 up to about 200 phr. Various activators such as zinc oxide, stearic acid, and the like, can also be used in amounts up to about 15 or more phr. Various antidegradants, and the like, well known in the art, can also be utilized. Such materials are generally mixed into the rubber by utilizing a mill, a Banbury mixer, or the like.

The rubber compositions can be used in a large number of applications, including finished articles such as tires.

The 4-pyrimidine sulfenamides of the present invention when utilized as primary accelerators with rubber have been found to yield very much improved cure rates and cure states, i.e., lower t25-t2 values, lower t90-t2 values, higher Vmax values, and higher Rmax values. The improved cure rate values were generally superior to the values obtained utilizing 2-pyrimidine sulfenamides or conventional thiazole sulfenamide primary accelerators such as N-cyclohexyl-2-benzothiazole sulfenamide, N-t-butyl-2-benzothiazole sulfenamide, N-t-butyl-2-benzothiazole sulfenimide and the like. Another unexpected result was that improved scorch delay was obtained for the 4-pyrimidine sulfenamides. However, it is also found to be advantageous to use the accelerators of the invention as auxiliary accelerators, in combination with other wellknown conventional accelerators, which include the guanidines, such as diphenylguanidine (DPG) or di-ortho-tolylguanidine (DOTG), the various thiazoles, such as 2-mercaptobenzothiazole and 2,2'-benzothiazole disulfide; benzothiazole sulfenamides, such as N-cyclohexyl-2-benzothiazole sulfenamide, N,N-dicyclohexyl-2-benzothiazole sulfenamide, N,N-diethyl-2-benzothiazole sulfenamide. N,N-diisopropyl-2-benzothiazole sulfenamide, N-oxydiethylene-2-benzothiazole sulfenamide, N-isopropyl-2-benzothiazole sulfenamide and N-t-butyl-2-benzothiazole sulfenamide. When EPDM rubber is vulcanized, a thiazole accelerator is generally used in combination with a thiuram accelerator. Examples of conventional thiuram accelerators include N,N'-dimethyl-N,N'-diphenylthiuram disulfide, dipentamethylenethiuram hexasulfide, tetramethylthiuram monosulfide, tetraethylthiuram disulfide, tetrabutylthiuram disulfide,
tetramethylthiuram disulfide, and metal salts of the corresponding dithiocarbamic acids, such as those of zinc, copper, tellurium, etc.

From 0.1 to 0.5 phr of the accelerators of the invention can be used, together with larger amounts (from 0.2 to 2.0 phr) of one or more conventional accelerators. Conversely, a small (0.1 to 0.5 phr) amount of one or more conventional accelerators can be used with a larger amount of one of the accelerators of the invention.

The invention will be better understood by reference to the following examples in which all parts are by weight and all temperatures are in degrees Celsius, unless otherwise specified.

## EXAMPLES

Various 4-pyrimidine sulfenamides of the present invention were tested in accordance with appropriate ASTM procedures for rubber. Parameters which characterize vulcanization were taken from ODR (oscillating disc rheometer) cure curves ("rheographs"), which were obtained for vulcanization at 153°. As is graphically shown in the drawing, the parameters Rmin and Rmax are the minimum rheometer torque (before the onset of vulcanization) and the maximum rheometer torque (due to vulcanization), respectively. The parameter t2 is the time required for an increase (over Rmin) in rheometer torque of 2.2dNm (2.0 in-lb); t25 is the time required for the occurrence of 25 percent of the increase in torque due to vulcanization (time at which torque equals (Rmax-Rmin)0.25 + Rmin) ; t90 is the time required for the occurrence of 90 percent of the increase in torque due to vulcanization (time at which torque equals (Rmax-Rmin)0.9 + Rmin). Vmax is the maximum slope of the vulcanization curve divided by Rmax-Rmin and expressed in terms of percent per minute.

The invention will be better understood by reference to the following examples in which all parts are per 100 parts by weight of rubber (phr) and all temperatures are in degrees Celsius, unless otherwise specified.

### Preparation of Rubber Masterbatches for Accelerator Evaluation

The various examples of 4-pyrimidine sulfenamide accelerators which were prepared were tested in typical NR and SBR carbon-black reinforced compounds.

An SBR rubber masterbatch was prepared, based on SBR-1500, containing the following ingredients:

| SBR Masterbatch | Parts |
|---|---|
| SBR-1500 | 100.0 |
| Carbon Black N-330 | 50.0 |
| Circosol 4240, a Naphthenic Oil, ASTM D2226, Type 103 | 10.0 |
| Zinc Oxide | 4.0 |
| Stearic Acid | 2.0 |
| | 166.0 |

The SBR masterbatch was prepared by mixing the above-noted components in a Banbury mixer according to standard techniques. Subsequently, various accelerators, sulfur, and an antidegradant were added on a laboratory roll mill in the amounts set forth hereinbelow and blended by using standard laboratory mill mixing techniques:

| | Parts |
|---|---|
| SBR-Masterbatch | 166.0 |
| SANTOFLEX 13 | 2.0 |
| Sulfur | 2.0 |
| Accelerators | As indicated |

SBR-1500 is a cold emulsion-polymerized, non-pigmented styrene/butadiene copolymer rubber containing nominally 23.5 percent bound styrene;

SANTOFLEX® 13 is N-(1,3-dimethylbutyl)-N'-phenyl-para-phenylenediamine, an antidegradant.

In a similar manner, a natural rubber masterbatch was made:

| Natural Rubber Masterbatch | Parts |
|---|---|
| Natural Rubber (SMR-CV) | 100.0 |
| Carbon Black N-330 | 50.0 |
| Naphthenic Oil; Circosol 4240 | 5.0 |
| Zinc Oxide | 5.0 |
| Stearic Acid | 2.0 |
| Total | 162.0 |

The natural rubber masterbatch was blended with the following compounds according to standard laboratory mill-mixing techniques:

| | Parts |
|---|---|
| Natural Rubber Masterbatch | 162.0 |
| SANTOFLEX 13 | 2.0 |
| Sulfur | 2.5 |
| Accelerators | As indicated |

The following Examples 1-4 show the preparation of a number of 4-pyrimidine sulfenamides of the invention together with control compounds outside the scope of the invention. Following this are Tables I-III which set forth the test data for rubber compositions of the invention (and the control compositions).

EXAMPLE 1

N-t-Butyl-4-pyrimidine Sulfenamide

4-Mercaptopyrimidine (2.24g, .02 moles) was transferred to an 100 ml 3-necked reaction flask equipped with a mechanical stirrer, and t-butylamine (30 ml) was added. An exothermic reaction with salt formation occurred and the slurry was allowed to stir overnight under a drying tube. An aqueous solution of sodium hypochlorite (10.6 g., 15.4%) was then added to the amine salt slurry by means of a dropping funnel. Intermittent water bath cooling was used to maintain the reaction temperature at less than 35°C during the approximately 15 minute addition period. The reaction mixture was stirred for 3 hours at room temperature and poured into deionized water (150 ml). After stirring for a few minutes, the product was collected by filtration and washed with two portions of water. It was suction dried and allowed to stand in the hood overnight. The product was a white solid (3.1 g, 85%, m.p. 106-108°C). Its purity was confirmed by LC and NMR: (δ, multiplicity, assignment, integration) 8.8-7.2, m, aromatic, 3 H; 1.8, s, NH, 1 H; 1.2, s, t-butyl, 9 H.

EXAMPLE 2 (Control: isomeric comparison for EXAMPLE 1) N-t-Butyl-2-pyrimidine Sulfenamide

2-Mercaptopyrimidine (6.72 g) was stirred with t-butylamine (63 ml) for one hour at room temperature in a 3-neck reaction flask equipped with mechanical stirrer and thermometer. Then a solution of sodium hypochlorite in water (31.8 g, 15.4%) was added over a 30 minute period at 30-40°C. The reaction mixture was stirred for three hours at room temperature, poured into 450 ml of deionized water, and the mixture stirred thoroughly and stored overnight in the refrigerator.

The aqueous suspension was then extracted twice with 200 ml portions of methylene chloride. The combined extracts were washed two times with an equal volume of deionized water, dried with anhydrous sodium sulfate, and then evaporated under reduced pressure. The product was a yellow oil (5.0 g) which crystallized to a solid, m.p. 60-66°C, upon standing at room temperature. The product was shown to be pure by L.C. analysis and NMR spectroscopy: (δ, multiplicity, assignment, integration) 8.8-7.2, m, aromatic, 3 H; 3.7, s, NH, 1 H; 1.6, s, t-butyl, 9 H.

EXAMPLE 3

N-t-Butyl-2,6-dimethyl-4-pyrimidine Sulfenamide

2,6-Dimethyl-4-mercaptopyrimidine (3.4 g, .024 moles) was stirred with t-butylamine (37 ml) in a 3-necked round bottom flask equipped with mechanical stirrer, dropping funnel, and thermometer. A solution of sodium hypochlorite (11.56 g, 17.2%) in water was then added over a 30 minute period at a temperature less than 35°C. Water bath cooling was used to maintain the temperature. After stirring for three hours at room temperature, the reaction mixture was stirred with deionized water (175 ml), but no solid separated. The reaction mixture was extracted twice with methylene chloride (200 ml portions). The combined organic extracts were washed with water, dried with anhydrous sodium sulfate, and evaporated under reduced pressure. The crude liquid product was only about 60% pure by liquid chromatographic analysis. Pure material (m.p. 65-71°C) was obtained by recrystallization from hexane, and as a residue product upon trituration with hexane. It was a single component by LC analysis and pure by NMR: (δ, multiplicity, assignment, integration) 7.3, s, aromatic, 1 H; 3.0, s, NH, 1 H; 2.6, s, methyl, 3 H; 2.4, s, methyl, 3 H; 1.2, s, t-butyl, 9 H.

EXAMPLE 4 (Control: isomeric comparison for EXAMPLE 3)

N-Cyclohexyl-4,6-dimethyl-2-pyrimidine Sulfenamide

This compound was prepared via the method outlined in Example 4 of U. S. Patent 3,839,303.

TABLE I

| COMPOUNDS OF EXAMPLES 1 and 2 IN SBR | | |
|---|---|---|
| Run # | 1 | 2 |
| SBR Masterbatch | 166 | 166 |
| Compound Ex. 1 | 1.2 | - |
| Compound Ex. 2 | - | 1.2 |
| Mooney Scorch, | | |
| 135°,$t_5$,min. | 41.9 | 44.9 |
| ODR Data @ 153° | | |
| Rmax, Nm | 5.06 | 4.49 |
| Rmin, Nm | 0.56 | 0.56 |
| t90, min. | 23.7 | 32.8 |
| t2, min. | 12.8 | 14.2 |
| t90-t2, min. | 10.9 | 18.6 |
| t25, min. | 17.0 | 18.5 |
| t25-t2, min. | 4.2 | 4.3 |
| Max. Veloc. of | | |
| vulc., %/min. | 24.6 | 14.0 |

TABLE II

| COMPOUNDS OF EXAMPLES 1 and 2 IN NATURAL RUBBER | | |
|---|---|---|
| Run # | 3 | 4 |
| NR Masterbatch | 162 | 162 |
| Compound Ex. 1 | 0.6 | - |
| Compound Ex. 2 | - | 0.6 |
| Mooney Scorch, | | |
| 120°, t5, min. | 35.9 | 44.7 |
| ODR Data @ 153° | | |
| Rmax, Nm | 4.38 | 4.04 |
| Rmin, Nm | 0.37 | 0.37 |
| t90, min. | 8.9 | 13.0 |

TABLE II (continued)

| COMPOUNDS OF EXAMPLES 1 and 2 IN NATURAL RUBBER | | |
|---|---|---|
| t2, min. | 3.8 | 4.7 |
| t90-t2, min. | 5.0 | 8.3 |
| t25,min. | 5.0 | 6.1 |
| t25-t2, min. | 1.2 | 1.4 |
| Max. veloc. of vulc, %/min. | 37.7 | 22.6 |

**TABLE III**

**Relative* Comparison of Compounds of Examples 3 and 4 in SBR and Natural Rubber**

| Run # | 5 | 6 | 7 | 8 | 9 | 10 |
|---|---|---|---|---|---|---|
| SBR Masterbatch | 166.0 | 166.0 | 166.0 | - | - | - |
| NR Masterbatch | - | - | - | 162.0 | 162.0 | 162.0 |
| TBBS | 1.2 | - | - | 0.6 | - | - |
| Compound Ex. 3 | - | 1.2 | - | - | 0.6 | |
| Compound Ex. 4 | - | - | 1.2 | - | - | 0.6 |
| Mooney Scorch, t5, min. 135°C | 1.00 | 2.17 | 1.99 | 1.00 | 1.35 | 1.06 |
| ODR Data @ 153°C | | | | | | |
| Rmax | 1.00 | 1.05 | 0.89 | 1.00 | 1.09 | 0.92 |
| Rmin. | 1.00 | 0.96 | 0.99 | 1.00 | 0.95 | 1.04 |
| t90 | 1.00 | 1.51 | 2.20 | 1.00 | 0.77 | 1.35 |
| t2 | 1.00 | 1.54 | 1.70 | 1.00 | 0.98 | 1.07 |
| t90-t2 | 1.00 | 1.48 | 2.62 | 1.00 | 0.64 | 1.57 |
| t25 | 1.00 | 1.64 | 1.63 | 1.00 | 1.00 | 1.12 |
| t25-t2 | 1.00 | 1.92 | 1.41 | 1.00 | 1.08 | 1.31 |
| Max. veloc. of vulc. | 1.00 | 1.28 | 0.54 | 1.00 | 1.88 | 0.61 |

* Relative defined as experimental values for runs #6 and 7 divided by these values for run #5; 9 and 10 divided by 8.

The test results set forth in Tables I, II and III show the effectiveness with the different compounds of the invention. In all instances, the 4-pyrimidine compounds of the invention gave better (faster) cure rates and higher extents of cure than the 2-pyrimidine controls (which are outside the scope of the invention). Thus, it is a surprising and unexpected result that moving the sulfur-bearing moiety from the 2-position to the 4-position has a dramatic effect on cure rate and cure state (extent of cure).

## Claims

1. A composition comprising sulfur-vulcanizable rubber and from 0.1 to 10 parts by weight per 100 parts by weight of rubber of a compound of the formula

PmSNRR'

wherein Pm is 4-pyrimidyl, optionally substituted on the nucleus by one or more halogen atoms or lower alkyl, phenyl, lower alkoxy or hydroxyl groups; R is H, or $C_{1-8}$ alkyl, $C_{3-8}$ cycloalkyl, phenyl, $C_{7-12}$ aralkyl or $C_{7-12}$ alkaryl; R' is H or R, or R and R' together with N form a heterocyclic ring.

2. The composition of Claim 1 wherein R is isopropyl, t-butyl or cyclohexyl and R' is H.

3. The composition of Claim 1 wherein R' is H.

4. The composition of Claim 1 wherein Pm is unsubstituted.

5. The composition of Claim 2 wherein Pm is unsubstituted.

6. The composition of Claim 1 also comprising from 0.1 to 5 parts by weight of sulfur, from 5 to 200 parts by weight of a filler and from 0.1 to 5 parts by weight of an antidegradant per 100 parts by weight of rubber.

7. The composition of Claim 6 also comprising from 5 to 200 parts by weight of oil.

8. The composition of Claim 2 wherein R is t-butyl.

9. The composition of Claim 2 wherein R is isopropyl.

10. The composition of Claim 2 wherein R is cyclohexyl.

11. The composition of Claim 7, also including from 0.1 to 0.5 phr of a conventional accelerator.

12. The composition of Claim 7 wherein the compound is present in an amount of from 0.1 to 0.5 parts by weight, and a conventional accelerator is also present, in an amount of from 0.2 to 2.0 parts by weight, per 100 parts by weight of rubber.

13. A compound of the formula

PmSNRR'

wherein Pm is 4-pyrimidyl, optionally substituted on the nucleus by one or more halogen atoms or lower alkyl, phenyl, lower alkoxy or hydroxy groups; R is $C_{1-8}$ alkyl, $C_{3-8}$ cycloalkyl, phenyl, $C_{7-12}$ alkaryl or $C_{7-12}$ aralkyl, and R' is R or H.

14. A compound of Claim 13 wherein R is isopropyl, t-butyl or cyclohexyl, and R' is H.

15. A compound of Claim 13 wherein Pm is unsubstituted.

16. N-t-Butyl-4-pyrimidine sulfenamide.

17. N-t-Butyl-2,6-dimethyl-4-pyrimidine sulfenamide.

18. N-Isopropyl-4-pyrimidine sulfenamide.

19. N-isopropyl-2,6-dimethyl-4-pyrimidine sulfenamide.

**20.** N-Cyclohexyl-4-pyrimidine sulfenamide.

**21.** N-Cyclohexyl-2,6-dimethyl-4-pyrimidine sulfenamide.

**Patentansprüche**

**1.** Zusammensetzung, umfassend schwefelvulkanisierbaren Kautschuk und 0,1 bis 10 Masseteile, pro 100 Masseteile Kautschuk, einer Verbindung der Formel

PmSNRR'

worin Pm 4-Pyrimidyl, gegebenenfalls substituiert am Nucleus durch ein oder mehrere Halogenatome oder nied. Alkyl-, Phenyl-, nied.Alkoxy- oder Hydroxyl-Gruppen, bedeutet; R H oder $C_1$-$C_8$-Alkyl, $C_3$-$C_8$-Cycloalkyl, Phenyl, $C_7$-$C_{12}$-Aralkyl oder $C_7$-$C_{12}$-Alkaryl darstellt; R' H oder R ist, oder R und R' zusammen mit N einen heterocyclischen Ring bilden.

**2.** Zusammensetzung nach Anspruch 1, worin R Isopropyl, tert.Butyl oder Cyclohexyl bedeutet, und R' H darstellt.

**3.** Zusammensetzung nach Anspruch 1, worin R' die Bedeutung H hat.

**4.** Zusammensetzung nach Anspruch 1, worin Pm unsubstituiert ist.

**5.** Zusammensetzung nach Anspruch 2, worin Pm unsubstituiert ist.

**6.** Zusammensetzung nach Anspruch 1, welche auch 0,1 bis 5 Masseteile Schwefel, 5 bis 200 Masseteile eines Füllstoffs und 0,1 bis 5 Masseteile eines Anti-Abbaumittels, pro 100 Masseteile Kautschuk, umfaßt.

**7.** Zusammensetzung nach Anspruch 6, welche auch 5 bis 200 Masseteile Öl umfaßt.

**8.** Zusammensetzung nach Anspruch 2, worin R tert.Butyl bedeutet.

**9.** Zusammensetzung nach Anspruch 2, worin R Isopropyl darstellt.

**10.** Zusammensetzung nach Anspruch 2, worin R Cyclohexyl ist.

**11.** Zusammensetzung nach Anspruch 7, welche auch 0,1 bis 0,5 phr eines herkömmlichen Beschleunigers enthält.

**12.** Zusammensetzung nach Anspruch 7, worin die Verbindung in einer Menge von 0,1 bis 0,5 Masseteilen vorliegt, und auch ein herkömmlicher Beschleuniger in einer Menge von 0,2 bis 2,0 Masseteilen, pro 100 Masseteile Kautschuk, anwesend ist.

**13.** Verbindung der Formel

PmSNRR'

worin Pm 4-Pyrimidyl, gegebenenfalls substituiert am Nucleus durch ein oder mehrere Halogenatome oder nied. Alkyl-, Phenyl-, nied.Alkoxy- oder Hydroxy-Gruppen, bedeutet; R $C_1$-$C_8$-Alkyl, $C_3$-$C_8$-Cycloalkyl, Phenyl, $C_7$-$C_{12}$-Alkaryl oder $C_7$-$C_{12}$-Aralkyl darstellt; und R' R oder H ist.

**14.** Verbindung nach Anspruch 13, worin R Isopropyl, tert.Butyl oder Cyclohexyl bedeutet, und R' H darstellt.

**15.** Verbindung nach Anspruch 13, worin Pm unsubstituiert ist.

**16.** N-tert.Butyl-4-pyrimidinsulfenamid.

**17.** N-tert.Butyl-2,6-dimethyl-4-pyrimidinsulfenamid.

**18.** N-Isopropyl-4-pyrimidinsulfenamid.

**19.** N-Isopropyl-2,6-dimethyl-4-pyrimidinsulfenamid.

**20.** N-Cyclohexyl-4-pyrimidinsulfenamid.

**21.** N-Cyclohexyl-2,6-dimethyl-4-pyrimidinsulfenamid.

## Revendications

**1.** Composition comprenant un caoutchouc vulcanisable par le soufre et de 0,1 à 10 parties en poids, pour 100 parties en poids de caoutchouc, d'un composé de formule

PmSNRR'

dans laquelle Pm représente un groupe 4-pyrimidyle, dont le noyau porte éventuellement, en tant que substituant, un ou plusieurs atomes d'halogène ou groupes alkyle inférieur, phényle, alkoxy inférieur ou hydroxy, R représente un atome d'hydrogène ou un groupe alkyle en $C_{1-8}$, cycloalkyle en $C_{3-8}$, phényle, aralkyle en $C_{7-12}$ ou alkaryle en $C_{7-12}$, et R' représente un atome d'hydrogène ou un groupe R, ou bien R et R' forment un hétérocycle avec l'atome d'azote.

**2.** Composition conforme à la revendication 1, dans laquelle R présente un groupe isopropyle, t-butyle ou cyclohexyle, et R' représente un atome d'hydrogène.

**3.** Composition conforme à la revendication 1, dans laquelle R' représente un atome d'hydrogène.

**4.** Composition conforme à la revendication 1, dans laquelle le groupe Pm ne porte pas de substituant.

**5.** Composition conforme à la revendication 2, dans laquelle le groupe Pm ne porte pas de substituant.

**6.** Composition conforme à la revendication 1, qui contient aussi, pour 100 parties en poids de caoutchouc, de 0,1 à 5 parties en poids de soufre, de 5 à 200 parties en poids d'une charge et de 0,1 à 5 parties en poids d'un agent anti-dégradation.

**7.** Composition conforme à la revendication 6, qui contient aussi de 5 à 200 parties en poids d'une huile.

**8.** Composition conforme à la revendication 2, dans laquelle R représente un groupe t-butyle.

**9.** Composition conforme à la revendication 2, dans laquelle R représente un groupe isopropyle.

**10.** Composition conforme à la revendication 2, dans laquelle R représente un groupe cyclohexyle.

**11.** Composition conforme à la revendication 7, qui contient également de 0,1 à 0,5 partie d'un accélérateur classique, pour 100 parties en poids de caoutchouc.

**12.** Composition conforme à la revendication 7, dans laquelle il y a de 0,1 à 0,5 partie en poids de composé, et dans laquelle il y a également de 0,2 à 2,0 parties en poids d'un accélérateur classique, pour 100 parties en poids de caoutchouc.

**13.** Composé de formule

PmSNRR'

dans laquelle Pm représente un groupe 4-pyrimidyle, dont le noyau porte éventuellement, en tant que substituant, un ou plusieurs atomes d'halogène ou groupes alkyle inférieur, phényle, alkoxy inférieur ou hydroxy, R représente un groupe alkyle en $C_{1-8}$ , cycloalkyle en $C_{3-8}$, phényle, aralkyle en $C_{7-12}$ ou alkaryle en $C_{7-12}$, et R' représente un atome d'hydrogène ou un groupe R.

**14.** Composé conforme à la revendication 13, dans lequel R représente un groupe isopropyle, t-butyle ou cyclohexyle, et R' représente un atome d'hydrogène.

**15.** Composé conforme à la revendication 13, dans lequel le groupe Pm ne porte pas de substituant.

**16.** N-t-butyl-pyrimidine-4-sulfénamide.

**17.** N-t-butyl-2,6-diméthyl-pyrimidine-4-sulfénamide.

**18.** N-isopropyl-pyrimidine-4-sulfénamide.

**19.** N-isopropyl-2,6-diméthyl-pyrimidine-4-sulfénamide.

**20.** N-cyclohexyl-pyrimidine-4-sulfénamide,

**21.** N-cyclohexyl-2,6-diméthyl-pyrimidine-4-sulfénamide.

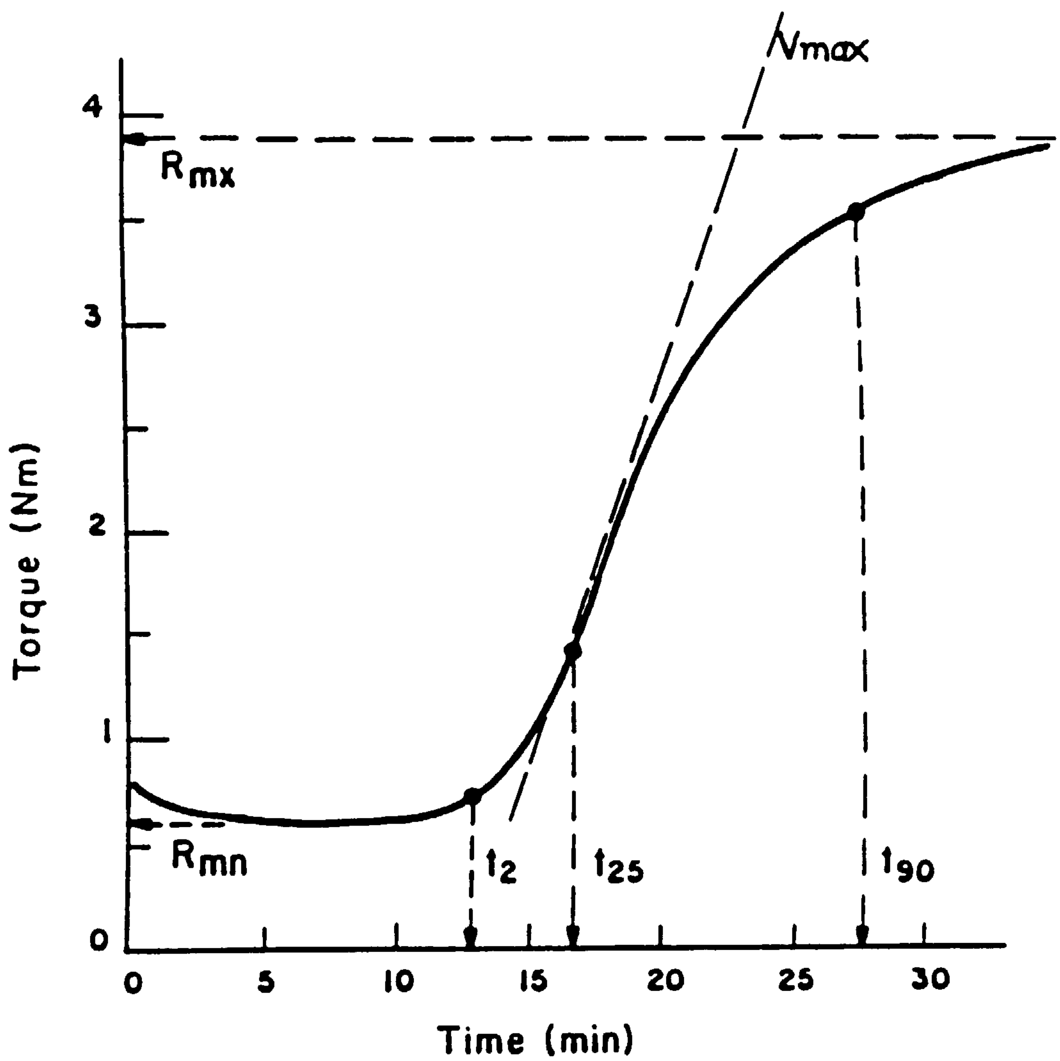


VULCANIZATION PARAMETERS